# EUROPEAN PATENT APPLICATION

(11) **EP 4 071 686 A1**
(43) Date of publication of application: **12.10.2022**
(21) Application number: 21382299.2
(22) Date of filing: 08.04.2021
(51) Int. Cl.: G06Q 10/10, G16H 10/40

(54) **LABORATORY SAMPLE DELIVERY AND BROKER SYSTEM**

(71) Applicant: F. Hoffmann-La Roche AG, 4070 Basel (CH); Roche Diagnostics GmbH, 68305 Mannheim (DE)
(72) Inventor: PAERSINNEN, Janne-Henrik, 1831 Mechelen (BE); VERCAMMEN, Werner, 1831 Mechelen (BE); VIEIRA PEDROSO, Nuno Miguel, 08174 Barcelona (ES); CORMANO BEL, Hector, 08174 Barcelona (ES); VILLACE DIEZ, Mario, 08174 Barcelona (ES)
(74) Representative: Herren, Barbara

(57) **Abstract**

A method for delivering medical samples with a digital laboratory request from an extraction point (230, 330) to a testing laboratory (100, 200, 120, 220, 320) is presented. The method comprises checking the availability of a test to be performed on a medical sample during a certain time period at a first testing laboratory (100, 200). If the test cannot be performed during the certain time period at the first testing laboratory (100, 200), laboratory attributes available at a plurality of testing laboratories (100, 200, 120, 220, 320) are checked via a plurality of laboratory broker devices (110, 125, 225, 325) communicatively connected by a communication network (115). A suitable candidate testing laboratory can be identified amongst the plurality of testing laboratories (100, 200, 120, 220, 320) based on the laboratory attributes of the suitable candidate testing laboratory. The identified suitable candidate testing laboratory and the first testing laboratory (100, 200) communicate with each other regarding the feasibility of the suitable candidate testing laboratory to perform the test on the medical sample. If feasible to perform the test at the suitable candidate testing laboratory, the medical sample and the digital laboratory request are delivered to the suitable candidate testing laboratory for testing. After performance of the test at the suitable candidate testing laboratory, the laboratory test results are communicated back to the extraction point (230, 330) in digital form.

## Description

### Technical Field

The present disclosure generally relates to a laboratory broker system for delivering medical samples to testing laboratories.

### Background

Typically, after a medical sample is extracted from a patient at a general practitioner's office, a courier picks up the medical sample to deliver it a centralized testing laboratory. The courier normally takes the same route to deliver the medical sample to the same centralized testing laboratory location. The courier usually does not try to optimize time and resources to deliver the medical samples to the centralized testing laboratory. Therefore, the medical samples may not be delivered to the most suitable laboratory or the laboratory with the most availability to test that medical sample.

Additionally, if a required test cannot be performed on the medical sample in the centralized testing laboratory after the courier delivers the medical sample, the medical sample may have to wait for the required test to become available and the test results may be delayed. Therefore, in the case of time- or temperature-sensitive tests, the quality of the test results may be compromised.

Therefore, there is a need for medical samples to be redirected/delivered to the closest laboratory with the most availability at the time the medical sample is picked up from the medical practice by the courier.

### Summary

The use of a laboratory broker component within a laboratory is proposed. This laboratory broker component can connect to other laboratory broker components in a network of testing laboratories in order to share data such as, for example, number of test order pending for a particular test at a particular testing laboratory, i.e., the availability of a particular test at a particular testing laboratory. In one embodiment, the network of testing laboratories can be a private laboratory chain. Such private laboratory chains typically have testing laboratories tailored to the needs of a particular area and the testing laboratories can be scattered throughout a geographical region in order to cover as much area as possible to reduce transportation costs. In another embodiment, the network of testing laboratories can be public testing laboratories in a public healthcare system. It can also be possible for the laboratory broker component can also take into consideration the number of laboratories available in the private laboratory chain or the public healthcare system.

If a particular test cannot be performed at a particular testing laboratory, the laboratory broker component can communicate with other laboratory broker components at the other testing laboratories in the network to determine if the other testing laboratories may possibly have availability to perform that particular test that cannot be performed at the original testing laboratory.

The other testing laboratories in the network that have availability to perform the test can post their key selling points of their laboratories to the original testing laboratory via the laboratory broker component system. Such key selling points can be, for example, geographical local of the testing laboratory, travel time for the sample to be transported to the testing laboratory, test cost/price at the testing laboratory, test capacity of the testing laboratory (i.e., number of tests performed per day), turn-around time (TAT) of test results from the laboratory, time to receive test results, quality control (QC) type used, QC precision and accuracy versus other testing laboratories' QC precision and accuracy, Interlaboratory QC programs, Six Sigma scores, Pre-analytic instrument accreditations, Post-analytic instrument accreditations, and/or validation type.

Upon identification of a suitable candidate testing laboratory, the corresponding laboratory broker components start communication between each other. If it is determined that it is feasible for the candidate testing laboratory to perform the required test, the test order and the medical sample are sent to that candidate laboratory. Both the test request and results are sent independently of the laboratory solution. Integrating the Healthcare Enterprise (IHE) Pathology and Laboratory Medicine (PaLM) domain can define the Inter Laboratory Workflow (ILW) profile that can be used for the interaction.

Additionally, the laboratory broker component can use its own proprietary communication logic, which can then be the same communication logic as all of the other laboratory broker components. IHE Palm domain can define the Inter Laboratory Codes Sets Distribution (LSCD) profile that can be used to share common nomenclatures such as, for example, available battery and test and observations codes, among the different laboratory broker components involved in this communication logic.

A method for delivering medical samples with a digital laboratory request from an extraction site to a testing laboratory is also presented. The method can comprise checking the availability of a test to be performed on a medical sample during a certain time period at a first testing laboratory, i.e., whether that test can be performed on the medical sample within that time period. If the test cannot be performed during the certain time period at the first testing laboratory, laboratory attributes available at a plurality of testing laboratories can be checked via a plurality of laboratory broker components located at the different testing laboratories communicatively connected by a communication network. A suitable candidate testing laboratory can then be identified amongst the plurality of testing laboratories based on the laboratory attributes of the suitable candidate testing laboratory. The suitable candidate testing laboratory and the first testing laboratory communicate regarding the feasibility of the suitable candidate testing laboratory to perform the test on the medical sample. If it is feasible to perform the test at the suitable candidate testing laboratory, the medical sample and the digital laboratory request are delivered to the suitable candidate testing laboratory for testing. After performance of the test on the medical sample at the suitable candidate testing laboratory, the laboratory test results are digitally communicated to the extraction site

If, however, it is not feasible to perform the test at the suitable candidate testing laboratory, the laboratory attributes available at the remaining testing laboratories in the plurality of testing laboratories can be rechecked via the plurality of laboratory broker components located in the testing laboratories in the network.

The plurality of laboratory broker components communicate using a shared common nomenclature and communication standard such as, for example, HL7 v2.5 Chapter 13: Clinical Laboratory Automation or HL7 FHIR Release 4: Diagnostic Medicine module. The shared common nomenclature and commination standard use the Interoperability profiles defined in the IHE PaLM domain such as, for example, Inter Laboratory Workflow (ILW) or Laboratory Code Sets Distribution (LCSD). ILW supports the workflow of orders and results with a subcontracting laboratory. LCSD distributes managed sets of clinical laboratory codes such as, for example, battery, test and observation codes. The plurality of laboratory broker components may also dynamically communicate over the communication network with the administration and reimbursement systems associated with the testing of the medical samples.

The checking of availability of a test to be performed on a medical sample during a certain time period at a first testing laboratory can occur in a delivery transport when the medical samples are picked up from the extraction site using a portable laboratory broker component installed on a personal portable device such as, for example, a smart phone or tablet.

The laboratory attributes of the testing laboratory can comprise at least one of the following: geographical local of the testing laboratory, time to the testing laboratory, test cost/price at the testing laboratory, test capacity of the testing laboratory (i.e., the number of tests performed per day), time to receive test results, quality control (QC) type used, QC precision and accuracy versus other laboratories' QC precision and accuracy, inter-laboratory QC programs, Six Sigma scores, Pre-analytic instrument accreditations, Post-analytic instrument accreditations, and/or validation type.

The feasibility of the suitable candidate-testing laboratory can also be based on future forecasted capacity at the suitable candidate-testing laboratory.

The centralized testing laboratory may also be able to validate medically and/or give medical advice regarding the test results depending on certificates and rights of that centralized testing laboratory.

A system to deliver medical samples from a first point to a testing laboratory is also presented. The system comprises a first laboratory broker component and a plurality of laboratory broker components. Each laboratory broker component can be resident in a testing laboratory. The system also comprises a communication network communicatively connected to the first laboratory broker component and the plurality of laboratory broker components. The first laboratory broker component and the plurality of laboratory broker components are configured to communicate laboratory attributes over the communication network to determine an available testing laboratory suitable to test the samples.

The first laboratory broker component can be a mobile application installed on a portable smart device or tablet device.

The first laboratory broker component is portable and located on delivery transport of the medical samples.

The first laboratory broker component can operate fully automatically in some embodiments.

In other embodiments, the first laboratory component can be configured with pre-existing conditions required of suitable laboratory attributes. The pre-existing conditions are geographic location, quality control type used, and/or quality control validation.

The first point can be a Point-of-Care (POC) device, a laboratory, a medical clinic, or a doctor's office.

The system, further comprises, an external database connected to the plurality of laboratory broker components configured to collect usage patterns of the plurality of laboratory broker components. The usage patterns comprise number of tests performed and reagents consumed at the testing laboratory.

There are several advantages of using the laboratory broker component system. One such advantage is that if for some reason the required tests become unavailable at the original testing laboratory, another testing laboratory can be found using the laboratory broker component and the medical sample can promptly be transported by courier to that suitable testing laboratory and the required test results can be obtained.

Secondly, the laboratory broker components system can be used to quickly find testing laboratories that have the specialized testing that can only be performed in specific testing laboratories reducing transporting needs and costs as well as making the testing of the medical sample more efficient.

Thirdly, some medical samples can be dynamically subcontracted to other testing laboratories in the network depending on the required tests and the workload of the original testing laboratory, thereby increasing the efficiency in testing. Additionally, the administration and reimbursement associated with the testing of the medical samples may also be dynamically allocated by the laboratory broker components system, which may also increase the efficiency for these allocations.

Fourthly, if abnormal test results are returned from a particular testing instrument device in a testing laboratory, i.e., raising doubts as to the quality of those test results, the medical sample can be couriered to another testing laboratory in the network and the required tests can be run again to double check the original test results, thereby, increasing the accuracy and validity of the test results of the medical sample.

Fifthly, by determining the best suited testing laboratory to run the required tests on a medical sample, the logistics of the transport system can be optimized and unnecessary work can be avoided such as, for example, unpacking, sorting, re-packing, and re-transporting the medical sample if the medical sample needs to be couriered to more than one testing laboratory, thereby, saving time and reducing costs.

Sixthly, if a testing laboratory is having technical difficulties, e.g., there is a power outage at the testing laboratory, the courier will be made aware via the laboratory broker component to avoid that testing laboratory until the technical difficulties have be resolved, e.g., the power has been restored to the testing laboratory. Further, a courier could also avoid a testing laboratory if it is forecasted that testing laboratory will not have the capacity to perform the required tests at the time of delivery of the medical sample.

Finally, depending of the urgency on receiving the results of the required test on a medical sample, the transportation route of the courier can be changed as well as the order of which the medical samples are dropped-off at the testing laboratories could be altered to take into consideration the urgency of the testing.

In another embodiment, not only can the laboratory broker optimize the delivery of medical samples to testing laboratories but the laboratory broker can also facilitate the delivery of laboratory consumables necessary to perform the tests on the medical samples to the testing laboratories throughout a laboratory network. Such laboratory consumables can be any consumable product used by a testing laboratory. Examples of laboratory consumables can be reagents and calibrators and/or sample tubes, sample wells, or pipette tips and/or even sample containers or transport boxes and the like.

### Brief Description of the Several Views of the Drawings

The following detailed description of specific embodiments of the present disclosure can be best understood when read in conjunction with the following drawings, where like structure is indicated with like reference numerals and in which:
Fig. 1 illustrates a laboratory brokerage system utilizing an external database for data collection according to an embodiment of the present disclosure.
Fig. 2 illustrates a laboratory brokerage system according to another embodiment of the present disclosure.
Fig. 3 illustrates a laboratory brokerage system with a portable laboratory broker according to an embodiment of the present disclosure.
Fig. 4 illustrates schematically a flowchart of the method of delivering medical samples from an extraction point to a testing laboratory according to an embodiment of the present disclosure.

### Detailed Description

In the following detailed description of the embodiments, reference is made to the accompanying drawings that form a part hereof, and in which are shown by way of illustration, and not by way of limitation, specific embodiments in which the disclosure may be practiced. It is to be understood that other embodiments may be utilized and that logical, mechanical and electrical changes may be made without departing from the spirit and scope of the present disclosure.

Certain terms will be used in this patent application, the formulation of which should not be interpreted to be limited by the specific term chosen, but as to relate to the general concept behind the specific term

The use of the 'a' or 'an' are employed to describe elements and components of the embodiments herein. This is done merely for convenience and to give a general sense of the inventive concepts. This description should be read to include one or at least one and the singular includes the plural unless it is obvious that it is meant otherwise.

The terms 'sample', 'patient sample' and 'biological sample' can refer to material(s) that may potentially contain an analyte of interest. The patient sample can be derived from any biological source, such as a physiological fluid, including blood, saliva, ocular lens fluid, cerebrospinal fluid, sweat, urine, stool, semen, milk, ascites fluid, mucous, synovial fluid, peritoneal fluid, amniotic fluid, tissue, cultured cells, or the like. The patient sample can be pretreated prior to use, such as preparing plasma from blood, diluting viscous fluids, lysis or the like. Methods of treatment can involve filtration, distillation, concentration, inactivation of interfering components, and the addition of reagents. A patient sample may be used directly as obtained from the source or used following a pretreatment to modify the character of the sample. In some embodiments, an initially solid or semi-solid biological material can be rendered liquid by dissolving or suspending it with a suitable liquid medium. In some embodiments, the sample can be suspected to contain a certain antigen or nucleic acid.

The term 'analyte' can relate to a component of a medical sample to be analyzed, e.g., molecules of various sizes, ions, proteins, metabolites and the like. Information gathered on an analyte may be used to evaluate the impact of the administration of drugs on the organism or on particular tissues or to make a diagnosis. Thus, 'analyte' can be a general term for substances for which information about presence, absence and/or concentration is intended. Examples of analytes are e.g., glucose, coagulation parameters, endogenic proteins (e.g., proteins released from the heart muscle), metabolites, nucleic acids and so on.

The term 'laboratory instrument' as used herein can encompass any apparatus or apparatus component operable to execute and/or cause the execution of one or more processing steps /workflow steps on one or more biological samples and/or one or more reagents. The expression 'processing steps' thereby can refer to physically executed processing steps such as centrifugation, aliquotation, sample analysis and the like. The term 'instrument' can cover pre-analytical instruments, post-analytical instruments, analytical instruments and laboratory middleware.

The term 'post-analytical instrument' as used herein can encompass any apparatus or apparatus component that can be configured to perform one or more post-analytical processing steps/workflow steps comprising - but not limited to - sample unloading, transport, recapping, decapping, temporary storage/ buffering, archiving (whether refrigerated or not), retrieval and/or disposal.

The term 'pre-analytical instrument' as used herein can encompass any apparatus or apparatus component that can be configured to perform one or more pre-analytical processing steps/workflow steps comprising - but not limited to - centrifugation, resuspension such as, for example, by mixing or vortexing, capping, decapping, recapping, sorting, tube type identification, sample quality determination and/or aliquotation steps. The processing steps may also comprise adding chemicals or buffers to a sample, concentrating a sample, incubating a sample, and the like.

The term 'analyzer' / 'analytical instrument' as used herein can encompass any apparatus or apparatus component configured to obtain a measurement value. An analyzer can be operable to determine via various chemical, biological, physical, optical or other technical procedures a parameter value of the sample or a component thereof. An analyzer may be operable to measure the parameter of the sample or of at least one analyte and return the obtained measurement value. The list of possible analysis results returned by the analyzer comprises, without limitation, can be concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent-holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow can be optimized for certain types of analysis. Examples of such analyzer can be clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'analysis or 'analytical test' as used herein can encompass a laboratory procedure in order to characterize a parameter of a biological/medical sample for qualitatively assessing or quantitatively measuring the presence or amount or the functional activity of an analyte.

The term 'reagent' as used herein can refer to materials necessary for performing an analysis of analytes, including reagents for sample preparation, control reagents, reagents for reacting with the analyte to obtain a detectable signal, and/or reagents necessary for detecting the analyte. Such reagents may include reagents for isolating an analyte and/or reagents for processing a sample and/or reagents for reacting with an analyte to obtain a detectable signal and/or washing reagents and/or diluents.

The terms 'sample container', 'sample holder' and 'sample tube' can refer to any individual container for storing, transporting, and/or processing a medical sample. In particular, the term without limitation refers to a piece of laboratory glass- or plastic-ware optionally comprising a cap on its upper end. The container comprising an opening for dispensing/ aspirating liquid into respectively out of the vessel. The opening may be closed by a cap, a breakable seal or like suitable means for closing the opening in a liquid-tight manner. Sample tubes, e.g., sample tubes used to collect blood, often comprise additional substances such as clot activators or anticoagulant substances, which have an impact on the processing of the sample. As a consequence, different tube types typically can be adapted for pre-analytical and analytical requirements of a particular analysis, e.g., a clinical chemistry analysis, a hematological analysis or a coagulation analysis. A mix up of sample tube types can make samples unusable for analysis. To prevent errors in the collection and handling of samples, the sample caps of many tube manufacturers are encoded according to a fixed and uniform color scheme. Some sample tubes types in addition or alternatively are characterized by particular tube dimensions, cap dimensions, and/or tube color. A dimension of a tube comprises e.g., its height, its size and/or further characteristic shape properties.

A 'data storage unit' or 'database' can be a computing unit for storing and managing data such as a memory, hard disk or cloud storage. This may involve data relating to biological/medical sample(s) to be processed by the automated system. The data management unit may be connected to an LIS (laboratory information system) and/or an HIS (hospital information system). The data management unit can be a unit within or co-located with a laboratory instrument. It may be part of the laboratory middleware. Alternatively, the database may be a unit remotely located. For instance, it may be embodied in a computer connected via a communication network.

The term 'communication network' as used herein can encompass any type of wireless network, such as a WiFi^{™}, GSM^{™}, UMTS or other wireless digital network or a cable based network, such as Ethernet^{™} or the like. In particular, the communication network can implement the Internet protocol (IP). For example, the communication network can comprise a combination of cable-based and wireless networks.

The term 'remote system' or 'server' as used herein can encompass any physical machine or virtual machine having a physical or virtual processor, capable of receiving; processing and sending data. A server can run on any computer including dedicated computers, which individually can also often be referred to as 'the server' or shared resources such as virtual servers. In many cases, a computer can provide several services and have several servers running. Therefore, the term server may encompass any computerized device that shares a resource with one or more client processes. Furthermore, the terms 'remote system' or 'server' can encompass a data transmission and processing system distributed over a data network (such as a cloud environment).

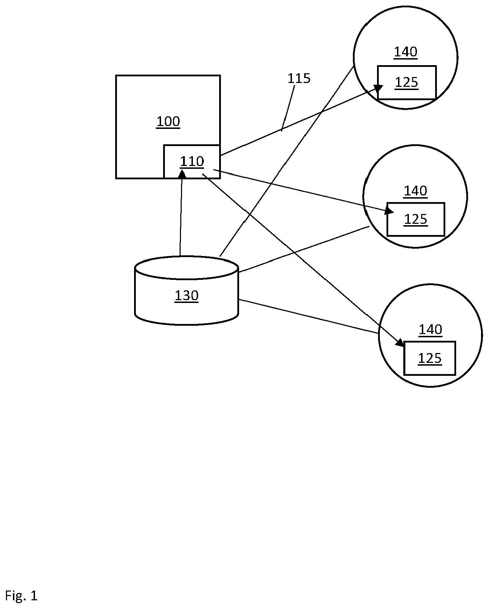

Referring initially to Fig. 1, a laboratory brokerage system (10) is illustrated. The laboratory brokerage system (10) comprises a plurality of testing laboratories (100, 120, 140). Each of the testing laboratories in the plurality comprises a laboratory broker component (110, 125). Each laboratory broker component (110, 125) communicates with each other via a communication network (115).

In one embodiment, a medical sample is extracted from a patientand delivered to a first testing laboratory, i.e., a centralized testing laboratory (100), within the plurality of testing laboratories (100, 120, 140). The extraction point (not shown) can be a doctor's office or a medical clinic, for example. In another embodiment, the medical sample is extracted from the patient at the centralized testing laboratory (100) itself. After receipt of the medical sample, the centralized testing laboratory (100) can determine if the centralized testing laboratory (100) has the capacity or capability to fulfill the test order on the received medical sample in the time required for accurate test results to be acquired.

If it is determined the test cannot be performed during the certain time period at the centralized testing laboratory (100), laboratory attributes available at the plurality of testing laboratories (120, 140) are checked via a plurality of laboratory broker components (125) communicatively connected to the laboratory broker component (110) of the centralized testing laboratory (100) by a communication network (115).

The laboratory attributes checked by the laboratory broker component (110) comprise at least one of the following: geographical location of the testing laboratory, time to get to the testing laboratory, available transportation to the laboratory, test cost/price, test capacity of the testing laboratory (i.e., number of tests per day), turn-around time (TAT) of test results from the laboratory, time to receive test results for the testing laboratory, quality control (QC) type used, QC precision and accuracy versus other laboratories' QC precision and accuracy, inter-laboratory QC programs, Six Sigma scores, Pre-analytic instrument accreditations, Post-analytic instrument accreditations, and/or validation type.

A suitable candidate testing laboratory (140) can then be identified amongst the plurality of testing laboratories (120, 140) based on the specific laboratory attributes of the suitable candidate testing laboratory (140). For example, each of the testing laboratories in the plurality of testing laboratories (120, 140) can be ranked according to the specific laboratory attributes that each testing laboratory possesses, i.e., the testing laboratory possessing the most laboratory attributes would be ranked the highest. The suitable candidate testing laboratory (140) and the centralized testing laboratory (100) then communicate via the communication network (115) regarding the feasibility of the suitable candidate testing laboratory (140) to perform the test on the medical sample in the required time.

If it is feasible to perform the test at the suitable candidate testing laboratory (140), the medical sample and the digital laboratory request are delivered to the suitable candidate testing laboratory (140) for testing. The medical sample and the digital laboratory request can be delivered to the suitable candidate testing laboratory 140 via a courier or any other known transportation delivery service. After performance of the test on the medical sample at the suitable candidate testing laboratory 140, the laboratory test results can be digitally communicated via the communication network (115) to the centralized testing laboratory (100) or to the original extraction point.

However, if it is determined that it is simply not feasible to perform the required test at the suitable candidate testing laboratory (140), the laboratory attributes available at the remaining testing laboratories (120) in the plurality of testing laboratories (120) are rechecked via the plurality of laboratory broker components (110,125) until another potential suitable testing laboratory is identified. Alternatively, the testing laboratory ranked the next highest can be contacted until a suitable candidate testing laboratory (140) is identified.

The plurality of laboratory broker components (110, 125) communicate over the communication network (115) using a shared common nomenclature and communication standard such as, for example, HL7 v2.5 Chapter 13: Clinical Laboratory Automation or HL7 FHIR Release 4: Diagnostic Medicine module. The shared common nomenclature and commination standard use the Interoperability profiles defined in the IHE PaLM domain such as, for example, Inter Laboratory Workflow (ILW) or Laboratory Code Sets Distribution (LCSD). ILW supports the workflow of orders and results with a subcontracting laboratory. LCSD distributes managed sets of clinical laboratory codes such as, for example, battery, test and observation codes. The plurality of laboratory broker components (110, 125) may also dynamically communicate over the communication network (115) with the administration and reimbursement systems associated with the testing of the medical samples.

In one embodiment, the centralized testing laboratory (100) or the suitable candidate testing laboratory (140) may be able to validate medical test results and/or give medical advice regarding the test results depending on certificates and rights of that centralized testing laboratory (100).

In one embodiment, the laboratory broker components (110, 125) can be fully automatic. In another embodiment, the laboratory broker components (110, 125) can be configured with some pre-existing constraints as to which testing laboratories (120) the laboratory broker components (125) may consider. For example, some pre-existing constraints can be geographical location of the testing laboratory, quality control type used by the testing laboratory, and validation type used by the testing laboratory.

In one embodiment, an external database 130 may be connected to all of the laboratory broker components (110, 125) in order to collect information regarding tests performed and statistics related to the performed tests from all of testing laboratories (100, 120) in the communication network (115). The collected information can then be used, for example, for learning purposes such as, for example, test usage and reagent consumption at the testing laboratories and/or development of new commercial offers.

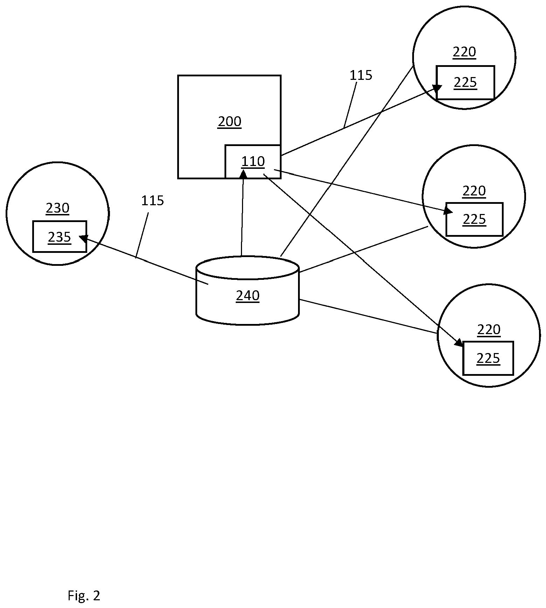

Fig. 2 illustrates an embodiment in which the extraction site (230) can be a Point-of-Care (POC) device, a laboratory, or any type of extraction site of the medical sample from a patient. In this embodiment, the extraction site (230) refers to the first site handling the sample tube containing the medical sample.

This extraction site (230) may not be a testing laboratory in the traditional sense of the word. Instead, the extraction site (230) can be a doctor's office such as, for example, a General Practitioner's office, an emergency clinic, a POC device such as, for example, a glucose meter, or any type of extraction site where a medical sample can be extracted from a patient. At this extraction point (230), some basic tests may be performed on the medical sample and, thus, in some cases, the medical sample may not need to be transported to a testing laboratory (200, 220) for further testing.

However, in this embodiment, if the medical sample is extracted from the patient at the extraction site (230) needs additional testing, the medical sample can be transported to a centralized testing laboratory (200) via courier or by any other means of medical sample transport. In one embodiment, the extraction site (230) may have its own laboratory broker component (235) which can be used to communicate via the communication network (115) to the laboratory broker components (110, 225) housed in the testing laboratories (200, 220) in the network. Alternatively, the extraction site (230) may not have its own laboratory broker component (235). In this case, the laboratory broker component (110) at the centralized testing laboratory (200), upon receiving the medical sample from the extraction site (230), may be used to communicate with the plurality of laboratory broker components (225) at that the plurality of testing laboratories (220) to find a suitable testing laboratory.

Upon stoppage of testing of the medical sample at the extraction site (230), the laboratory broker component (235) at the extraction site (230) can start looking for other laboratory broker components (110, 225) at different laboratories (200, 220) based on specific criteria defined at the extraction site (230) or the medical sample can be transported to the centralized testing laboratory (200) and that laboratory broker component (110) can communicate with the other laboratory broker components (225) at different laboratories (220) . In either case, the specific criteria can be, for example, time to receive results from the testing laboratory, production capacity of the testing laboratory, maximum price of the test at the testing laboratory, pre-analytic analyzer instruments accreditations and post-analytic instrument analyzer accreditations, specific or country regulations, QC accuracy and Six Sigma results of the testing laboratory, reliability of the medical sample, and so on.

In this embodiment, an external database (240) may also be connected to all of the laboratory broker components (110, 225, 235) in order to collect information and data from the plurality of testing laboratories (200, 220) connected to the communication network (115) as described above for Fig. 1.

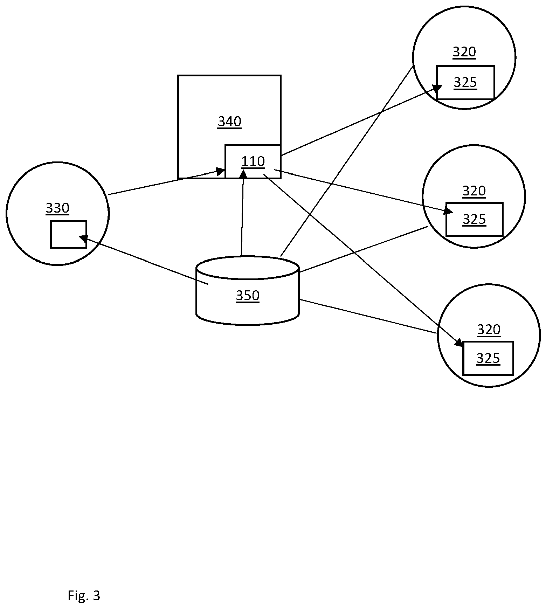

In Fig. 3, another embodiment of the laboratory broker component system is illustrated. In this embodiment, the laboratory broker component (110) may not be linked to/housed in a testing laboratory or extraction site at all. Instead, in this embodiment, the laboratory broker component (110) may be installed on a personal portable device (340) such as, for example, a smart phone or tablet of a courier. In other words, the portable laboratory component (110) can be an app downloaded and installed on a personal portable device of the courier. The portable laboratory broker component (110) may be carried by a courier in charge of transporting the medical sample to a suitable testing laboratory.

The courier may use the portable laboratory broker component (110) during transport of the medical samples from an extraction site (330), such as, for example, a doctor's office to the suitable testing laboratories (320). For example, as soon as the courier picks up a medical sample from the extraction site (330), the courier can use the portable laboratory broker component (110) to connect via the communication network (115) to all the testing laboratories (320) in that network in order to find the most suitable testing laboratory for performing the required tests on the medical sample.

In this embodiment, the portable laboratory broker component (110) can assign the final testing laboratory destination for the recently collected medical sample during transport of the medical ample without first having to transport the medical sample to a centralized testing laboratory location for distribution. The suitable testing laboratory can, therefore, be assigned in real time.

In one embodiment, the portable laboratory broker component (110) may be used to check to see which testing laboratory in the network of laboratories (320) is best suited, i.e., most optimal for each collected medical sample at a future delivery time such as, for example, 5 pm, by comparing, for example, the testing laboratory workloads, capabilities and the like. Thereby, the delivery site decision can be based on a future forecasted laboratory capacity at each of the laboratories in the network.

Again in this embodiment, an external database 350 may be connected to all of the laboratory broker components (110, 325) in order to collect information and data from the plurality of testing laboratories (330, 320) con the communication network (115) as was described above for Fig. 1.

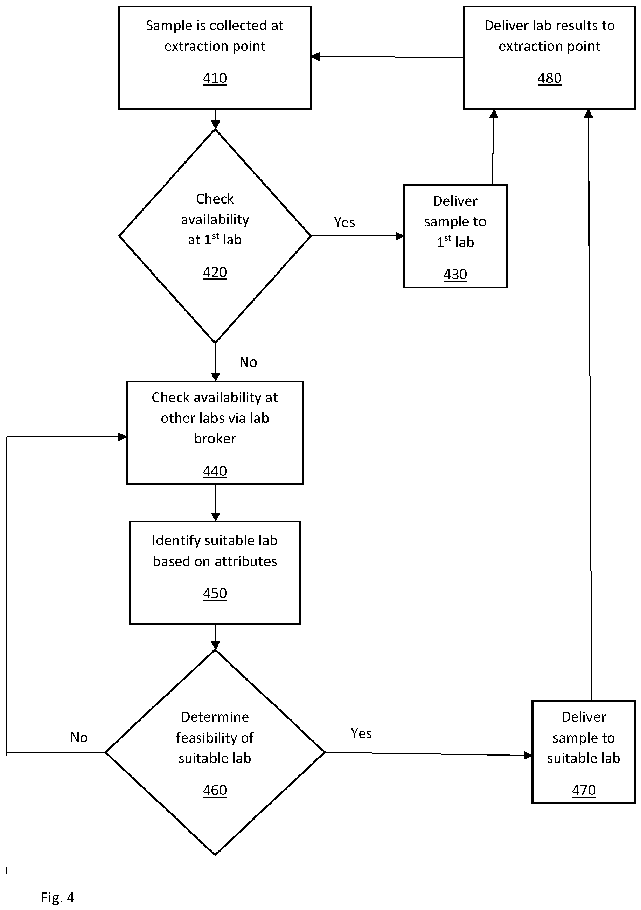

Fig. 4 illustrates, in summary, the overall method of delivering the medical sample from the extraction point (230, 330) to a testing laboratory (120, 220, 320). Once the medical sample is collected at the extraction point (230, 330) in step 410, the availability of the test that needs to be performed on the medical sample is assessed at the first testing laboratory (100, 200) in step 420. If the first testing laboratory (100, 200) is capable of performing the test, the medical sample and a digital laboratory request are transported to the first testing laboratory in step 430. If, on the other hand, the first testing laboratory (100, 200) cannot perform the test within the necessary testing parameters, the important laboratory testing attributes available at a plurality of testing laboratories (120, 220, 320) are checked via a plurality of laboratory broker components (110, 125, 225, 325) by a communication network (115) in step 440. In step 450, a suitable candidate-testing laboratory amongst the plurality of testing laboratories (120, 220, 320) is identified based on the laboratory attributes of the suitable candidate testing laboratory. In step 460, the feasibility of the suitable candidate-testing laboratory to perform the test on the medical sample is determined by communication between the suitable candidate-testing laboratory and the first testing laboratory (100, 200). If it is determined that it is feasible to perform the test at the suitable candidate testing laboratory, the medical sample and the digital laboratory request are delivered to the suitable candidate testing laboratory for testing in step 470. However, if it is not feasible to perform the test at the suitable candidate testing laboratory, the laboratory attributes are again rechecked, in step 440, at the remaining testing laboratories in the plurality of testing laboratories (120, 220, 330) via a plurality of laboratory broker components (110, 125, 225, 325). After performance of the test at the first testing laboratory (100, 200) or at the suitable candidate-testing laboratory, the laboratory test results are digitally communicated back to the extraction point (230, 330) in step 480.

It is noted that terms like "preferably," "commonly," and "typically" are not utilized herein to limit the scope of the claimed embodiments or to imply that certain features are critical, essential, or even important to the structure or function of the claimed embodiments. Rather, these terms are merely intended to highlight alternative or additional features that may or may not be utilized in a particular embodiment of the present disclosure.

Having described the present disclosure in detail and by reference to specific embodiments thereof, it will be apparent that modifications and variations are possible without departing from the scope of the disclosure defined in the appended claims. More specifically, although some aspects of the present disclosure are identified herein as preferred or particularly advantageous, it is contemplated that the present disclosure is not necessarily limited to these preferred aspects of the disclosure.

## Claims

1. A method for delivering medical samples along with a digital laboratory testing request from an extraction point (230, 330) to a testing laboratory (120, 220, 320), the method comprising:
checking availability of a test to be performed on a medical sample during a certain time period at a first testing laboratory (100, 200);
if the test cannot be performed during the certain time period at the first testing laboratory (100, 200), checking laboratory attributes available at a plurality of testing laboratories (120, 220, 320) via a plurality of laboratory broker components (110, 125, 225, 325) communicatively connected by a communication network (115);
identifying a suitable candidate testing laboratory amongst the plurality of testing laboratories (120, 220, 320) based on the laboratory attributes of the suitable candidate testing laboratory;
communicating between the suitable candidate testing laboratory and the first testing laboratory (100, 200) regarding the feasibility of the suitable candidate testing laboratory to perform the test on the medical sample;
if it is feasible to perform the test at the suitable candidate testing laboratory, delivering the medical sample and the digital laboratory request to the suitable candidate testing laboratory for testing; and
after performance of the test at the suitable candidate testing laboratory, digitally communicating laboratory test results to the extraction point (230, 330).

2. The method according to claim 1, further comprising,
if it is not feasible to perform the test at the suitable candidate testing laboratory, rechecking laboratory attributes available at the remaining testing laboratories in the plurality of testing laboratories (120, 220, 330) via a plurality of laboratory broker components (110, 125, 225, 325).

3. The method according to claims 1 and 2, wherein the plurality of laboratory broker components (110, 125, 225, 325) communicate using a shared common nomenclature and communication standard.

4. The method according to any one of the proceeding claims, wherein the shared common nomenclature and commination standard use the Interoperability profiles defined in the IHE PaLM domain.

5. The method according to any one of the proceeding claims, wherein the checking of availability of the test to be performed on the medical sample during the certain time period at the first testing laboratory (200) occurs in a delivery transport when the medical samples are picked up from the extraction point (230).

6. The method according to any one of the proceeding claims, wherein the laboratory attributes comprise at least one of the following: geographical local of the testing laboratories, time to get to the testing laboratory, cost/price of the test, test capacity of the testing laboratory (i.e., tests per day), time to receive test results, quality control (QC) type used at the testing laboratory, QC precision and accuracy versus other laboratories' QC precision and accuracy, inter-laboratory QC programs, Six Sigma scores, Pre-analytic instrument accreditations, Post-analytic instrument accreditations, and/or validation type.

7. The method according to any one of the proceeding claims, wherein feasibility of the suitable candidate testing laboratory is based on future forecasted capacity at the suitable candidate testing laboratory.

8. A system to deliver medical samples from a first point to a testing laboratory, the system comprising:
a first laboratory broker component (110);
a plurality of laboratory broker components (125, 225, 325), wherein each laboratory broker component is resident in a testing laboratory (100, 120, 220, 320); and
a communication network (115) communicatively connected to the first laboratory broker component (110) and the plurality of laboratory broker components (125, 225, 325), wherein the first laboratory broker component (110) and the plurality of laboratory broker components (125, 225, 325) are configured to communicate laboratory attributes over the communication network (115) to determine an available testing laboratory suitable to test the medical samples.

9. The system according to claim 8, wherein the first laboratory broker component (110) is a mobile application installed on a portable smart device (340).

10. The system according to claims 8 and 9, wherein the first laboratory broker component (110) is portable and located on delivery transport of the medical samples.

11. The system according to any one of the proceeding claims, wherein the first laboratory broker component (110) is configured with pre-existing conditions of suitable laboratory attributes.

12. The system according to any one of the proceeding claims, wherein the pre-existing conditions are geographic location of the testing laboratory, quality control type used at the testing laboratory, and/or quality control validation of the testing laboratory.

13. The system according to any one of the proceeding claims, wherein the first point (230, 330) is a Point-of-Care (POC) device, a testing laboratory, or a doctor's office.

14. The system according to any one of the proceeding claims, further comprising,
an external database (130) connected to the plurality of laboratory broker components (110, 125, 225, 325) configured to collect usage patterns from the plurality of laboratory broker components (110, 125, 225, 325) about the testing laboratories (100, 120, 220, 320).

15. The system according to claim 14, wherein the usage patterns comprise number of tests performed and reagents consumed at the testing laboratory.
